# EUROPEAN PATENT SPECIFICATION

(11) **EP 4 012 025 B1**
(45) Date of publication and mention of the grant of the patent: **23.09.2026**
(21) Application number: 21213792.1
(22) Date of filing: 10.12.2021
(51) Int. Cl.: C12Q 1/70, C12Q 1/6806, A01N 1/124, A01N 1/125

(54) **TRANSPORT MEDIUM FOR SAMPLES CONTAINING NUCLEIC ACIDS AND/OR PROTEINS**
TRANSPORTMEDIUM FÜR NUKLEINSÄUREN- UND/ODER PROTEINHALTIGE PROBEN
MILIEU DE TRANSPORT POUR LES ÉCHANTILLONS CONTENANT DES ACIDES NUCLÉIQUES ET/OU DES PROTÉINES

(30) Priority: 10.12.2020 CZ 20200665; 10.12.2020 CZ 202038335 U
(43) Date of publication of application: 15.06.2022
(73) Proprietor: Univerzita Palackého v Olomouci, 779 00 Olomouc (CZ); Aumed, a.s., 14300 Praha 4 (CZ)
(72) Inventor: Hajduch, Marian, 79305 Norbercany (CZ); Dzubak, Petr, 75103 Citov (CZ); Jaworek, Hana, 77900 Olomouc (CZ); Bouska, Ondrej, 53943 Otradov (CZ); Kubanova, Katerina, 70800 Ostrava (CZ); Vronka, Juraj, 16300 Praha 6 (CZ)
(74) Representative: Harber IP s.r.o.

(56) References cited:
- WO-A1-2018/027075
- WO-A1-97/31651
- CN-A- 103 169 964
- CN-A- 109 258 622
- RU-C1- 2 069 951
- RU-C1- 2 475 218
- US-A1- 2014 186 821
- TONGALP H. TEZEL ET AL: "Serum-free Media for Culturing and Serial-Passaging of Adult Human Retinal Pigment Epithelium", EXPERIMENTAL EYE RESEARCH, vol. 66, no. 6, 1 June 1998 (1998-06-01), pages 807 - 815, XP055211190, ISSN: 0014-4835, DOI: 10.1006/exer.1998.0492
- JIN T H ET AL: "Stabilizing formulations for inhalable powders of an adenovirus 35-vectored tuberculosis (TB) vaccine (AERAS-402)", VACCINE, ELSEVIER, AMSTERDAM, NL, vol. 28, no. 27, 17 June 2010 (2010-06-17), pages 4369 - 4375, XP027064056, ISSN: 0264-410X, [retrieved on 20100502], DOI: 10.1016/J.VACCINE.2010.04.059

## Description

### Field of Art

The present invention relates to a transport medium for the transport and storage of samples containing nucleic acids and/or proteins, in particular samples containing pathogens, for example viruses, and in particular SARS CoV-2 virus, but also host DNA and RNA and protein samples.

### Background Art

Nasopharyngeal or oropharyngeal swabs are currently used to examine the presence of SARS-CoV-2 virus (SARS, Wuhan 2019 CoV pneumonia virus) and other viruses invading the respiratory tract. The swab is performed with a synthetic swab, which is then placed in a test tube with a transport medium. Similarly, a transport medium is used to transport samples taken from other tissues intended for the diagnosis of viruses. A transport medium is also used for the transport of samples intended for the diagnosis of other pathogens, or of samples intended for the detection of the patient's DNA, RNA or proteins. The samples transported and stored in this way are typically used for nucleic acid isolation and subsequent detection (eg detection of viruses or other pathogens) by nucleic acid amplification (eg PCR or LAMP methods). The transport medium used is the so-called viral transport medium (VTM), which contains fetal bovine serum and optionally also serum albumin, as well as antibiotics and antifungals. A typical composition of VTM is, for example: 2% fetal bovine serum (FBS), 100 µg/ml gentamicin or another antibiotic; 0.5 µg/ml amphotericin B or another antifungal, Hanks's balanced salt solution (HBSS). The composition of the viral transport medium is not chemically definable and reproducible. Fetal bovine serum is a mixture containing thousands of proteins obtained from unborn calves. Thus, it is a product with a variable composition, which may additionally contain inhibitors of polymerization or reverse transcription, which further reduces the reproducibility of any amplification assays performed with nucleic acids from samples stored or transported in the VTM. Furthermore, the use of FBS greatly complicates or directly prevents the quantification of proteins in the VTM-transported or stored samples, as it is difficult to distinguish the sample proteins from the variable proportions of proteins present in FBS. Yet furthermore, the very procedure for obtaining fetal bovine serum is considered to be ethically problematic and requires the exploitation of animals, so it is desirable to replace FBS with other products wherever possible.

According to the manufacturers' data and the experience of the present inventors, biological samples taken at VTM are stable at 4 °C for up to 5-7 days, but their processing is recommended within a maximum of 3 days of collection/harvesting. Alternatively, samples may be transported in VTM in a frozen state, in which pathogens are stable for up to 6 months.

CN 109258622; RU 2475218; RU 2069951; Tezel et al: Experimental Eye Research, 1998, 807-815; US 2014/186821; CN 103169964; WO 97/31651; WO 2018/027075; Jin et al: Vaccine, 2010, 4369-4375 disclose various compositions for stabilizing biological samples, which comprise dextran as one of the components. However, these compositions are complex and contain components which are not precisely chemically defined, thereby limiting reproducibility.

The present invention aims to develop a well-chemically defined and reproducible transport medium for the transport and storage of nucleic acid and protein samples, which may include viruses and/or other pathogens, and/or host nucleic acids and/or proteins. Furthermore, this medium should be particularly suitable for the transport and storage of samples obtained by gargling (eg by self-sampling).

### Disclosure of the Invention

The present invention provides a transport medium consisting of a buffer solution selected from phosphate buffered saline and Hank's balanced salt solution, at least one antibiotic, at least one antifungal, and a polymer which is dextran.

Within the framework of the development of the novel transport medium, it was found that fetal bovine serum proteins can be replaced by dextran. The polymer must provide a suitable oncotic pressure, and a suitable balance of oncotic and osmotic pressure must also be ensured, in order to stabilize pathogens such as viruses as well as somatic cells in the medium. The combination of these parameters creates an environment similar to that of the organism in which the sample is stable.

Replacing proteins with suitable non-proteinaceous polymer which is dextran eliminates problems associated with the presence of proteins, especially with the presence of poorly defined protein mixtures such as FBS. Thus, no degradative enzymes are present in the medium which could degrade, destroy, or inhibit polymerization, amplification or, for example, reverse transcription in the downstream steps of the diagnostic or identification procedure. Proteins can also be easily detected or determined in a sample transported or stored in a dextran-containing medium. The medium has a clearly chemically defined composition, it is reusable with a stable and reproducible exact composition, and this leads to better reproducibility and lower error rate of all downstream methods.

Unlike standard VTMs, there is no need to use fetal bovine serum (FBS) in the transport medium of the present invention, its function being fully replaced by dextran. This also eliminates the ethical problems associated with the use of FBS, and also eliminates the need to use living animals, which is in line with the principles of the so-called 3Rs - replacement of live animals, reduction of the number of animals used, and refinement of the methods so as to cause the animals the lowest possible level of pain or stress. Dextran is therefore a complete replacement for a product whose production involves the use of animals.

Dextran is a polymer approved for pharmaceutical use as an excipient. The possible contact of the medium with the patient's organism is thus not risky and the polymer is well tolerated by various organisms.

Furthermore, with the framework of the present invention it was discovered that dextran stabilizes the biological material (in particular, viral material) even at room temperature, while not inhibiting nucleic acid amplification by PCR or LAMP. Stabilization at room temperature is a significant advantage over standard VTM.

The amount of dextran in the transport medium may preferably be in the range of 0.05 to 5% by weight, more preferably 0.1 to 2% by weight, even more preferably 0.1 to 1% by weight.

The relative molecular weight of dextran should be at least 10,000, for example in the range 10,000 to 100,000, more preferably 10,000 to 70,000. With a relative molecular weight of the polymer below 10,000, the required oncotic pressure may no longer be generated. The upper limit of the relative molecular weight of the polymer has no technical limitation.

The buffer solution in the transport medium of the invention is phosphate buffered saline (PBS) or Hanks's balanced salt solution (HBSS). More generally, the buffer solution is buffered saline.

Antibiotics and antifungals that are used in standard VTM can be used in the transport medium of the invention. The most commonly used antibiotics in VTMs are gentamicin, penicillin or streptomycin, but other antibiotics can be used as well. The most commonly used antifungal agent in VTM is amphotericin B, but other antifungal agents may be used as well. The antibiotic may preferably be present in an amount of 50 to 200 µg/ml. The antifungal may preferably be present in an amount of 0.1 to 2 µg/ml.

The medium of the present invention is well chemically defined, it does not contain a variable mixture of proteins. This medium has a stabilizing function even when storing samples at room temperature. At the same time, it allows seamless downstream detection of nucleic acids using nucleic acid amplification techniques, both qualitative and quantitative, including real-time modifications of such methods. It also allows easy and reliable protein detection.

The present invention further relates to the use of the above described medium for the transport and storage of samples containing nucleic acids or proteins, e.g. samples of pathogens or tissue or body fluid samples. Particularly preferably, these are samples taken from the patient by gargling, so-called gargling samples.

The present invention further relates to a method for detecting nucleic acid in a sample (taken from a patient's body), said method comprising a step of placing the sample into the medium according to the invention, a step of transporting and/or storing the sample in said medium and a step of determining the presence and/or the amount of a nucleic acid in the sample, using a PCR or LAMP technique.

The present invention further relates to a method for detecting a virus in a sample (taken from a patient's body), said method comprising a step of placing the sample into the medium according to the invention, a step of transporting and/or storing the sample in said medium and a step of determining the presence and/or the amount of the virus in the sample, using a PCR or LAMP technique.

The medium, similarly to a common VTM, is suitable for the transport and storage of viruses, both DNA and RNA viruses. In particular, the virus may be SARS-CoV-2, HPV and other human or animal diseases such as hepatitis, influenza, coronavirus, tick-borne encephalitis, smallpox, mononucleosis, HIV, etc. The medium is further suitable for transport and storage of other pathogens detectable by PCR, such as mycoplasmas, chlamydia, etc.

### Examples of carrying out the Invention

### Example 1: Selection of polymers

With regard to the possibility of contact of a patient with the components of the medium, polymers which have been used in pharmaceutical formulations and are therefore reliably harmless to health have been tested in particular.

Individual polymers were tested for inhibition of amplification by LAMP and PCR. Commercial kits AUMED test RT-LAMP Assay SARS-CoV-2 from AUMED, as, Prague, Czech Republic, Novel Coronavirus (2019-nCoV) Real Time Multiplex RT-OCR Kit (Detection 3 genes) from Liferiver, Shanghai ZJ Bio-Tech Co. were used for testing. The manufacturer's instructions were followed. The amplified sample was SARS-CoV-2 nucleic acid.

The results of this assay are shown in Table 1, which shows that only dextran and polyvinylpyrrolidone polymers did not inhibit LAMP amplification. However, dextran showed a better stabilization of samples in further tests.

**Table 1. LAMP inhibition assay by candidate polymers ***

| **Polymer** | SARS dilution 10⁻⁵/ 10⁻² / 0 | Concentration |
|---|---|---|
| SoluPlus | +/0/0 | 1.00 % |
| Lutrol F 68 | ++/0/0 | 1.00 % |
| Kollidon 17 PF | ++ /++ / **0** | 1.00 % |
| Kolliphor RH40 | ++/0/0 | 1.00 % |
| Dextran (MW 40000) | ++ / + / **0** | 1.00 % |
| Polyvinylpyrrolidone (MW 40000) | ++ / + / **0** | 1.00 % |
| Polyethylene glycol (MW 6000) | +/0/0 | 1µm |
| PCR water | +/0/0 | |
| Phosphate buffer saline (PBS) | +/0/0 | |

| | | |
|---|---|---|
| ++- highly positive, +- mildly positive, 0- negative; *- PCR reaction was not inhibited by the tested polymers. | | |

### Example 2: Preparing media for testing

Gentamicin stock solution at a final concentration of 100 µg/ml gentamicin, amphotericin B stock solution at a final concentration of 0.5 µg/ml amphotericin B, and dextran (relative molecular weight 40,000) were added to buffered saline (PBS) in the amount indicated in the individual tests (typically at a final concentration of dextran 0.1% by weight, 0.5% by weight, or 1% by weight). The media was used directly or stored at room temperature.

### Example 3: Stability testing of SARS-CoV-2 in media

In this experiment, the stability of SARS-CoV-2 in the media according to the invention was tested, compared to standard transport virological media. The medium according to the invention contained 0.1% by weight, 0.5% by weight or 1% by weight of dextran.

Stability was tested at laboratory temperature (24-27 °C) and at 4 °C for 15 days. On day 0, 6223 PFU/ml and 62.23 PFU/ml of SARS-CoV-2 virus were inoculated into each test medium. (PFU = plaque forming units). Only one replicate from each media alternative was tested under the given conditions.

Isolation of nucleic acid isolation was then performed using the RNA-VIRAL-PREP-96 kit (Palacky University Olomouc, IMTM) or VIRAL-RNA-Prep (ITA-Intertact). We used the Novel Coronavirus (2019-nCoV) Real Time Multiplex RT-PCR Kit (Liferiver, Shanghai ZJ Bio-Tech Co.) to detect coronaviruses. The test detects 3 genes, namely ORF1, gene N and gene E. Furthermore, an internal control (IC) was detected. Isolation and detection were performed according to the manufacturer's instructions. Tests were also performed with negative and positive controls; controls from the above commercial kit were used.

The results are shown in Table 2 below, the values given are in Ct. It was shown that the virological transport medium according to the invention stabilized the virus for 2 weeks, even at laboratory temperature or at 37 °C.

### Example 4: Stability testing of SARS-CoV-2 in media

In this experiment, the stability of SARS-CoV-2 in the media of the invention was tested, compared to standard transport virological media. Media with 0.5% by weight of dextran and comparative media containing additionally 0.25 % BSA were selected for this testing. Stability was tested at 4 °C, laboratory temperature (24 °C) and 37 °C for 15 days. On day 0, 62.23 PFU/ml SARS-CoV-2 was inoculated into each test medium. Three replicates from each medium alternative were tested under the given conditions.

Isolation of nucleic acids was performed using the RNA-VIRAL-PREP-96 kit (Palacky University Olomouc, IMTM) or VIRAL-RNA-Prep (ITA-Intertact). We used the Novel Coronavirus (2019-nCoV) Real Time Multiplex RT-PCR Kit (Liferiver, Shanghai ZJ Bio-Tech Co.) to detect coronaviruses. The test detects 3 genes, namely ORF1, gene N, gene E. Furthermore, an internal control (IC) was detected. Isolation and detection were performed according to the manufacturer's instructions.

The results are shown in Table 3 below, the values are given in Ct. It was shown that the viral transport medium according to the invention stabilized the virus for 2 weeks, even at laboratory temperature.

### Example 5: Stability testing of samples from SARS-CoV-2 gargling tests in media

To finally verify the stability of the sample taken in the medium containing 0.5% by weight of dextran, a simulated sample was tested. After gargling with 10 to 20 ml of drinking water for 10 to 30 s, the contents were poured into a collection vessel with lyophilized medium and, after closing, shaken thoroughly. After adding 10 to 20 ml of gargling, the sample in the collection vessel should contain approximately the same amount of media components as in previous stability tests. The simulated sample was created by mixing a gargling sample from three individuals. Stability was tested at 4 °C, room temperature (24 °C) and 37 °C for 15 days. On day 0, 62.23 PFU/ml SARS-CoV2 was inoculated into each test medium. Three replicates from each medium alternative were tested under the given conditions.

Isolation of nucleic acids was performed using RNA-VIRAL-PREP-96 (Palacky University Olomouc, IMTM) or VIRAL-RNA-Prep (ITA-Intertact). We used the Novel Coronavirus (2019-nCoV) Real Time Multiplex RT-PCR Kit (Liferiver, Shanghai ZJ Bio-Tech Co.) to detect coronaviruses. The test detects 3 genes, namely ORF1, gene N, gene E. Furthermore, an internal control (IC) was detected. Isolation and detection were performed according to the manufacturer's instructions.

The stability of SARS-CoV-2 for its subsequent detection by real-time PCR in pure medium containing 0.5% by weight of dextran and in a simulated real sample was demonstrated. The results are summarized in Table 4, the values are given in Ct.

**Table 2. SARS-CoV-2 detection stability assay in virological transport media containing dextran**

| medium | temp. (°C) | virus concentration (PFU/ml) | **Day 0** | | | | **Day 4** | | | | **Day 10** | | | | **Day 15** | | | |
|---|---|---|---|---|---|---|---|---|---|---|---|---|---|---|---|---|---|---|
| | | | **ORF1** | **N gene** | **E gene** | **IC** | **ORF1** | **N gene** | **E gene** | **IC** | **ORF1** | **N gene** | **E gene** | **IC** | **ORF1** | **N gene** | **E gene** | **IC** |
| DEX 1% | 4 | 6223 | 19.12 | 16.59 | 15.07 | 14.39 | 20.37 | 17.87 | 16.45 | 17.58 | 24.94 | 22.37 | 20.12 | 19.69 | 22.19 | 20.29 | 19.81 | 19.92 |
| DEX 1% | 24-27 | 6223 | 19.44 | 16.63 | 14.87 | 14.56 | 20.13 | 18.39 | 16.94 | 17.85 | 22.99 | 23.72 | 19.94 | 19.52 | 20.87 | 19.96 | 19.09 | 18.83 |
| DEX 0.5% | 4 | 6223 | 19.26 | 16.32 | 14.94 | 14.32 | 19.72 | 17.47 | 16.44 | 17.54 | 21.86 | 19.87 | 19.13 | 19.93 | 21.41 | 19.67 | 19.09 | 19.44 |
| DEX 0.5% | 24-27 | 6223 | 19.20 | 16.28 | 14.92 | 14.06 | 20.38 | 18.14 | 16.92 | 17.93 | 21.59 | 19.92 | 18.86 | 18.91 | 21.19 | 19.38 | 18.92 | 19.07 |
| DEX 0.1% | 4 | 6223 | 19.12 | 16.34 | 14.98 | 13.85 | 20.13 | 17.71 | 16.69 | 17.82 | 22.12 | 23.35 | 19.46 | 19.08 | 21.28 | 19.37 | 18.81 | 19.10 |
| DEX 0.1% | 24-27 | 6223 | 19.46 | 16.39 | 15.22 | 14.27 | 20.33 | 17.93 | 16.89 | 17.99 | 21.34 | 19.26 | 18.60 | 19.13 | 21.02 | 19.31 | 18.81 | 19.03 |
| DEX 1% | 4 | 62.23 | 27.18 | 24.93 | 23.74 | 21.40 | 30.95 | 29.85 | 27.84 | 27.98 | 30.94 | 29.18 | 28.50 | 28.78 | 31.06 | 29.52 | 29.01 | 29.11 |
| DEX 1% | 24-27 | 62.23 | 27.53 | 25.28 | 23.66 | 12.70 | 29.43 | 30.66 | 27.31 | 27.29 | 30.97 | 36.24 | 30.05 | 28.88 | 30.01 | 29.98 | 28.87 | 28.77 |
| DEX 0.5% | 4 | 62.23 | 27.73 | 24.68 | 23.47 | 21.63 | 30.59 | 28.28 | 26.93 | 27.89 | 32.30 | ND | 30.99 | 29.38 | 31.32 | 29.43 | 29.12 | 29.28 |
| DEX 0.5% | 24-27 | 62.23 | 27.76 | 24.98 | 23.54 | 21.56 | 29.71 | 27.61 | 26.42 | 27.36 | 30.57 | 29.42 | 28.47 | 28.05 | 29.49 | 27.68 | 27.47 | 27.81 |
| DEX 0.1% | 4 | 62.23 | 28.62 | 26.87 | 24.79 | 22.00 | 30.68 | 27.90 | 26.94 | 27.80 | 31.85 | 29.24 | 28.89 | 29.07 | 31.89 | 29.95 | 29.56 | 29.51 |
| DEX 0.1% | 24-27 | 62.23 | 28.74 | 25.99 | 24.42 | 20.37 | 28.99 | 26.41 | 25.00 | 26.71 | 30.99 | 28.93 | 28.40 | 28.36 | 30.88 | 29.52 | 29.10 | 28.98 |
| NTC_PCR | | | ND | ND | ND | ND | ND | ND | ND | ND | ND | ND | ND | ND | ND | ND | ND | ND |
| POZ_PCR | | | 26.38 | 24.88 | 23.86 | 23.18 | 26.44 | 24.86 | 24.33 | 25.88 | 28.10 | 27.48 | 26.99 | 28.32 | 26.86 | 26.54 | 26.37 | 26.77 |

| | | | | | | | | | | | | | | | | | | |
|---|---|---|---|---|---|---|---|---|---|---|---|---|---|---|---|---|---|---|
| DEX- dextran (MW 40000), IC- internal control, ND- not detected, NTC_PCR- PCR negative control, POZ_PCR- PCR positive control | | | | | | | | | | | | | | | | | | |

**Table 3. SARS-CoV-2 detection stability assay in virological transport media containing 0.5% dextran or 0.25% dextran with 0.5% BSA**

| Medium | temp. (°C) | virus concentration (PFU/ml) | **Day 0** | | | | **Day 8** | | | **Day 15** | | | | |
|---|---|---|---|---|---|---|---|---|---|---|---|---|---|---|
| | | | **ORF1** | **N gene** | **E gene** | **IC** | **ORF1** | **N gene** | **E gene** | **IC** | **ORF1** | **N gene** | **E gene** | **IC** |
| **DEX 0.5%** | 4 | 62.23 | 28 | 27.98 | 26.6 | 26.52 | 29.73 | 27.34 | 26.76 | 26.92 | 29.70 | 29.07 | 27.34 | 28.89 |
| **DEX 0.5%** | 4 | 62.23 | 27.91 | 27.69 | 26.66 | 26.56 | 29.47 | 27.21 | 26.78 | 26.83 | 30.06 | 29.67 | 27.85 | 28.77 |
| **DEX 0.5%** | 4 | 62.23 | 28.08 | 27.85 | 26.75 | 26.59 | 29.43 | 27.43 | 26.98 | 26.99 | 30.08 | 29.76 | 28.06 | 28.92 |
| **DEX 0.5%** | 24-27 | 62.23 | 27.56 | 27.92 | 26.73 | 26.59 | 29.19 | 27.33 | 26.81 | 27 | 29.44 | 29.38 | 27.43 | 28.63 |
| **DEX 0.5%** | 24-27 | 62.23 | 28.06 | 27.95 | 26.75 | 26.66 | 28.75 | 27.14 | 26.66 | 26.82 | 29.85 | 29.70 | 27.69 | 28.75 |
| **DEX 0.5%** | 24-27 | 62.23 | 27.8 | 27.56 | 26.11 | 25.96 | 29.51 | 27.51 | 26.85 | 26.95 | 30.16 | 30.22 | 28.12 | 28.72 |
| **DEX 0.5%** | 37 | 62.23 | 28 | 27.75 | 26.58 | 26.47 | 28.72 | 26.97 | 26.55 | 26.78 | 29.87 | 29.49 | 27.80 | 28.85 |
| **DEX 0.5%** | 37 | 62.23 | 27.8 | 28.13 | 26.82 | 26.63 | 28.52 | 27.24 | 26.77 | 26.97 | 29.95 | 29.65 | 27.70 | 28.71 |
| **DEX 0.5%** | 37 | 62.23 | 27.71 | 27.76 | 26.62 | 26.44 | 28.8 | 27.03 | 26.57 | 26.86 | 30.12 | 29.90 | 28.08 | 28.70 |
| **NTC_PCR** | | | ND | ND | ND | ND | ND | ND | ND | ND | ND | ND | ND | ND |
| **POZ_PCR** | | | 29.37 | 29.76 | 28.96 | 29.37 | 26.86 | 26.54 | 26.37 | 26.77 | 29.38 | 29.14 | 28.90 | ND |

| | | | | | | | | | | | | | | |
|---|---|---|---|---|---|---|---|---|---|---|---|---|---|---|
| DEX- dextran (MW 40000), IC- internal control, ND- not detected, NTC_PCR- PCR negative control, POZ_PCR- PCR positive control | | | | | | | | | | | | | | |

**Table 4. SARS-CoV-2 detection stability assay in simulated sample and in virological transport media containing 0.5% dextran**

| Medium | temp.(°C) | virus concentration (PFU/ml) | **Day 0** | | | | **Day 10** | | | | **Day 15** | | | |
|---|---|---|---|---|---|---|---|---|---|---|---|---|---|---|
| | | | **ORF1** | **N gene** | **E gene** | **IC** | **ORF1** | **N gene** | **E gene** | **IC** | **ORF1** | **N gene** | **E gene** | **IC** |
| **Simulated sample** | 4 | 62.23 | 28.30104 | 26.27271 | 24.93232 | 32.6511 | 27.72 | 27.3 | 25.61 | 25.32 | 27.4 | 26.64 | 26.08 | 26.62 |
| **Simulated sample** | 4 | 62.23 | 27.81531 | 25.96016 | 24.47147 | 31.80365 | 27.39 | 25.9 | 24.6 | 24.59 | 27.43 | 26.83 | 26.29 | 26.87 |
| **Simulated sample** | 4 | 62.23 | 27.57232 | 25.54524 | 24.28279 | 31.91608 | 26.73 | 25.35 | 23.87 | 23.95 | 27.58 | 26.86 | 26.2 | 26.8 |
| **Simulated sample** | 24-27 | 62.23 | 28.30497 | 26.33928 | 24.82027 | 30.03663 | 27.43 | 25.64 | 24.47 | 24.45 | 28.05 | 26.98 | 26.59 | 26.93 |
| **Simulated sample** | 24-27 | 62.23 | 28.44377 | 26.35123 | 25.06617 | 32.61991 | 27.23 | 25.75 | 24.5 | 24.49 | 28.96 | 27.64 | 26.94 | 27.38 |
| **Simulated sample** | 24-27 | 62.23 | 28.17863 | 25.98015 | 24.83246 | 31.53025 | 27.47 | 26.32 | 24.63 | 24.79 | 29.98 | 28.62 | 27.98 | 28.36 |
| **Simulated sample** | 37 | 62.23 | 28.21349 | 26.20653 | 24.67084 | 33.34145 | 28.78 | 27.32 | 26.23 | 25.68 | 31.87 | 30.07 | 29.66 | 29.6 |
| **Simulated sample** | 37 | 62.23 | 28.21293 | 26.24546 | 24.97092 | 31.11663 | 28.9 | 27.71 | 26.55 | 25.79 | 31.96 | 30.5 | 30.13 | 29.73 |
| **Simulated sample** | 37 | 62.23 | 29.36083 | 27.05949 | 25.80214 | 31.90566 | 28.64 | 26.94 | 25.72 | 25.8 | 29.76 | 28.44 | 28.08 | 28.23 |
| **DEX 0.5%** | 24-27 | 62.23 | 28.45057 | 25.36855 | 24.33951 | 31.70112 | 27.71 | 26.28 | 25.38 | 25.48 | 31.03 | 30.55 | 30.26 | 29.13 |
| **DEX 0.5%** | 24-27 | 62.23 | 28.85078 | 26.00466 | 24.45164 | 31.2165 | 27.88 | 26.63 | 25.72 | 25.6 | 31 | 30.6 | 30.3 | 29.2 |
| **DEX 0.5%** | 24-27 | 62.23 | 28.49731 | 25.62587 | 24.26845 | 31.02895 | 27.76 | 26.53 | 25.57 | 25.64 | 30.75 | 29.77 | 29.68 | 28.84 |
| **NTC_PCR** | | | ND | ND | ND | ND | ND | ND | ND | ND | ND | ND | ND | ND |
| **POZ_PCR** | | | 25.97505 | 24.93557 | 24.65564 | ND | 26.8 | 27.16 | 26.17 | 26.43 | 35.37 | 33.48 | 34.19 | ND |

### Example 6: Clinical validation study

The validation study was performed with a gargling assay which contains the virological transport medium of the present invention. The virological transport medium in the gargling test was prepared by preparing a liquid medium containing 0.5% by weight of dextran and lyophilizing this medium.

The test subjects performed a self-sampling by gargling with 10 to 20 ml of drinking water for 10 to 30 s, then the contents were spit out into a collection vessel with lyophilized medium and shaken thoroughly after sealing.

RNA isolation was performed after transport and possibly several days of storage of the collection vessel, using the isolation kit with paramagnetic beads VIRAL-RNA-Prep (ITA Intertact), and RT-PCR detection was performed using the Novel Coronavirus (2019-nCoV) Real Time Multiplex RT kit. -OCR Kit (Detection 3 genes) from Liferiver, Shanghai ZJ Bio-Tech Co.). The manufacturer's instructions were followed for isolation and detection.

The summary results of PCR detection are shown in the following table:

| PCR | | Sample | | Total |
|---|---|---|---|---|
| | | Positive | Negative | |
| | Positive | 206 | 0 | 206 |
| Gargling | Negative | 24 | 229 | 253 |
| | Total | 230 | 229 | 459 |

Based on the results of the assays, the following statistical parameters of the gargling test using the virological transport medium according to the invention were calculated (CI = confidence interval):
- sensitivity: SE = 89.6%, 95%CI = (84.9%, 93.2%)
- specificity: SP = 100.0%, 95%CI = (98.4%, 100.0%)
- negative predictive value: NPV = 90.5%, 95%CI = (86.2%, 93.8%)
- positive predictive value: PPV = 100.0%, 95%CI = (98.2%, 100.0%)
- accuracy = 94.8%, 95%CI = (92.3%, 96.6%).

### Example 7: Isolation of viral DNA from samples stored in the media.

In this experiment, the possibility of transporting and detecting DNA viruses, particularly HPV16 virus were studied. The medium according to the invention contained 0.5% by weight of dextran.

The SiHa HPV16 positive cell line was selected, and a different number of the cells (4 - 4.000.000) was used to test the sensitivity of the detection. The cells were counted and added to the transporting media. Only one replicate from each cell number was tested.

In parallel we have analysed two patient samples obtained in media by gargling with pure water and mixing with the lyophilised aliquote according to the invention contained 0.5% by weight of dextran.

Isolation of nucleic acid was then performed using the Zybio EXM6000 isolation system in just 9 minutes using the Zybio T-200-96 viral nucleic acid isolation system or manually by the QIAamp^{®} DNA detection by Micro kit (Qiagen, Hilden, Germany).

For the detection was used the PapilloCheck^{®} HPV-Screening kit (Greiner Bio-One, Frickenhausen, Germany). Isolation and detection were performed according to the manufacturer's instructions. Tests were also performed with negative and positive controls; controls from the above commercial kit were used.

The results are shown in Table 5 below, the values given are in Cp. It was shown that the virological transport medium is compatible with the isolation of the viral DNA from the cultivated cells and the real patient samples.

**Table 5. HPV16 detection assay in SiHa HPV16 positive cell line and two human samples in virological transport media containing 0.5% dextran**

| **Sample** | **Zybio isolation - Input** | | **HPV16** | **GAPDH** |
|---|---|---|---|---|
| | **Cell number** | **Copy number** | **Cp** | **Cp** |
| SiHa | 4000000 | 4000000 | 24,06 | 24,82 |
| SiHa | 400000 | 400000 | 25,03 | 25,5 |
| SiHa | 40000 | 40000 | 28,18 | 26,64 |
| SiHa | 4000 | 4000 | 32,37 | 27,41 |
| SiHa | 400 | 400 | 35,45 | 27,51 |
| SiHa | 40 | 40 | 39,75 | 27,29 |
| SiHa | 4 | 4 | - | 27,59 |
| Patient 1 | - | - | 35,78 | 26.34 |
| Patient 2 | - | - | 35,76 | 24.88 |
| GARG | - | - | - | 27,17 |

### Example 8: Isolation of genomic DNA from samples stored in the media.

In this experiment, the possibility of transporting and detecting genomic DNA was studied. The medium according to the invention contained 0.5 % by weight of dextran.

The patient samples were obtained in media by gargling with pure water and mixing with the lyophilised aliquote according to the invention containing 0.5 % by weight of dextran.

Isolation of nucleic acid was then performed using the Zybio EXM6000 isolation system in just 9 minutes using the Zybio T-200-96 viral nucleic acid isolation system.

For the detection was used the SARS-CoV-2 Nucleic Acid Detection Kit. As a part of the kit the GAPDH gene detection primers are available and this gene serves as the internal sampling control for each sample. Isolation and detection were performed according to the manufacturer's instructions. Tests were also performed with negative and positive controls; controls from the above commercial kit were used.

The results are shown in Table 6 below, the values are given in Cp. It was shown that the virological transport medium is compatible with the isolation of the genomic DNA from the real patient samples.

**Table 6. GAPDH detection assay in human samples in transport media containing 0.5% dextran**

| **Zybio isolation - input** | |
|---|---|
| **Name** | **GAPDH (Cp)** |
| Sample1 | 25,74 |
| Sample2 | 25,88 |
| Sample3 | 26,65 |
| Sample4 | 25,91 |
| Sample5 | 25,55 |
| Sample6 | 25,38 |
| Sample7 | 24,96 |
| Sample8 | 24,54 |
| Sample9 | 26,07 |
| Sample10 | 23,78 |
| Sample11 | 24,09 |
| Sample12 | 26,96 |
| Sample13 | 24,85 |
| Sample14 | 24,41 |
| Sample15 | 26,74 |
| Sample16 | 27,37 |
| Sample17 | 26,72 |
| Sample18 | 24,07 |
| Sample19 | 23,82 |
| Sample20 | 23,51 |
| Sample21 | 26,62 |
| Sample22 | 27,78 |
| Sample23 | 25,62 |
| Sample24 | 27,05 |
| Sample25 | 27,71 |
| Sample26 | 20,50 |
| Sample27 | 24,25 |
| Sample28 | 26,79 |
| Sample29 | 28,10 |
| Sample30 | 27,69 |
| Sample31 | 22,73 |
| Sample32 | 28,60 |
| Sample33 | 25,78 |
| Sample34 | 24,34 |
| Sample35 | 25,86 |
| Sample36 | 25,05 |
| POZ_PCR | 29,89 |
| NTC_PCR | - |

### Example 9: Isolation of proteins from samples stored in the media.

In this experiment, the possibility of isolating proteins from the human samples stored in the medium was studied. The medium according to the invention contained 0.5 % by weight of dextran.

The patient samples were obtained in media by gargling with pure water and mixing with the lyophilised aliquote according to the invention containing 0.5 % by weight of dextran. Isolation of proteins was then performed by the precipitation of the proteins by the modified method for the protein purification and extraction by TRI Reagent^{®} (SigmaAldrich).

The concentration of the isolated proteins was measured by the standard Bradford method. The results are shown in Table 7 below, the values given are in µg/ml. It was shown that the virological transport medium is compatible with the isolation of the proteins from the real patient samples.

**Table 7. Protein concentration in human samples in transport media containing 0.5% dextran**

| Name of sample | Protein concentration (µg/ml) |
|---|---|
| Patient 1 | 133,3 |
| Patient 2 | 50,4 |
| Patient 3 | 303,8 |
| Patient 4 | 152,8 |
| Patient 5 | 90,4 |

## Claims

1. Transport medium for transport and storage of samples containing nucleic acids and/or proteins, said medium consisting of a buffer solution selected from phosphate buffered saline and Hank's balanced salt solution, at least one antibiotic, at least one antifungal, and dextran.

2. Transport medium according to claim 1, wherein the amount of dextran in the liquid transport medium is in the range from 0.05 to 5% by weight.

3. Transport medium according to any one of claims 1 and 2, wherein the relative molecular weight of dextran is at least 10,000.

4. Use of the transport medium according to any one of claims 1 to 3 for the transport and/or storage of pathogen samples and/or tissue samples and/or body fluid samples.

5. Method for detecting nucleic acid in a sample taken from a patient's body, comprising the steps of
- contacting the collected sample with the transport medium according to any one of claims 1 to 3,
- transporting and/or storing the said sample in the transport medium, and
- subsequently determining by PCR or LAMP the presence and/or amount of a predetermined nucleic acid in the sample.

6. Method for detecting nucleic acid of a pathogen, in particular a virus, in a sample taken from mouth cavity and/or respiratory tract of a patient, comprising the steps of
- contacting the collected sample with the transport medium according to any one of claims 1 to 3,
- transporting and/or storing the said sample in the transport medium, and
- subsequently determining by a nucleic-acid-amplification-based method the presence and/or amount of a nucleic acid of the pathogen in the sample.

7. Use according to claim 4, or method according to claim 6, wherein the pathogen is a coronavirus, more preferably SARS-CoV-2 virus.

## Patentansprüche

1. Transportmedium zum Transport und zur Lagerung von Proben, die Nukleinsäuren und/oder Proteine enthalten, worin das Medium besteht aus einer Pufferlösung, ausgewählt aus phosphatgepufferter Salzlösung und Hank's Pufferlösung, mindestens einem Antibiotikum, mindestens einem Antimykotikum und Dextran.

2. Transportmedium nach Anspruch 1, wobei der Dextrangehalt im flüssigen Transportmedium zwischen 0,05 und 5 Gew.-% liegt.

3. Transportmedium nach einem der Ansprüche 1 oder 2, wobei die relative Molekülmasse des Dextrans mindestens 10.000 beträgt.

4. Verwendung des Transportmediums nach einem der Ansprüche 1 bis 3 zum Transport und/oder zur Lagerung von Pathogenproben und/oder Gewebeproben und/oder Körperflüssigkeitsproben.

5. Verfahren zum Nachweis von Nukleinsäure in einer aus dem Körper eines Patienten entnommenen Probe, umfassend die folgenden Schritte:
- Inkubieren der entnommenen Probe mit dem Transportmedium gemäß einem der Ansprüche 1 bis 3,
- Transportieren und/oder Lagern der Probe im Transportmedium und
- anschließende Bestimmung des Vorhandenseins und/oder der Menge einer vorbestimmten Nukleinsäure in der Probe mittels PCR oder LAMP.

6. Verfahren zum Nachweis von Nukleinsäure eines Pathogens, insbesondere eines Virus, in einer aus der Mundhöhle und/oder den Atemwegen eines Patienten entnommenen Probe, umfassend die folgenden Schritte:
- Inkubieren der entnommenen Probe mit dem Transportmedium gemäß einem der Ansprüche 1 bis 3,
- Transportieren und/oder Lagern der Probe im Transportmedium und
- anschließende Bestimmung des Vorhandenseins und/oder der Menge der Nukleinsäure des Pathogens in der Probe mittels eines Nukleinsäureamplifikationsverfahrens.

7. Verwendung nach Anspruch 4 oder Verfahren nach Anspruch 6, wobei es sich bei dem Pathogen um ein Coronavirus, vorzugsweise um das SARS-CoV-2-Virus, handelt.

## Revendications

1. Milieu de transport pour le transport et le stockage d'échantillons contenant des acides nucléiques et/ou des protéines, ledit milieu étant constitué d'une solution tampon choisie parmi le solution saline phosphatée et la solution saline équilibrée de Hank, d'au moins un antibiotique, d'au moins un antifongique et de dextrane.

2. Milieu de transport selon la revendication 1, où la quantité de dextrane dans le milieu de transport liquide est comprise entre 0,05 et 5 % en poids.

3. Milieu de transport selon l'une quelconque des revendications 1 et 2, où la masse moléculaire relative du dextrane est d'au moins 10 000.

4. Utilisation du milieu de transport selon l'une quelconque des revendications 1 à 3 pour le transport et/ou le stockage d'échantillons de pathogènes et/ou de tissus et/ou de liquides biologiques.

5. Procédé de détection d'acide nucléique dans un échantillon prélevé sur un patient, comprenant les étapes suivantes :
- mise en contact de l'échantillon prélevé avec le milieu de transport selon l'une quelconque des revendications 1 à 3 ;
- transport et/ou stockage dudit échantillon dans le milieu de transport ;
- ultérieurement, détermination par PCR ou LAMP de la présence et/ou de la quantité d'un acide nucléique prédéterminé dans l'échantillon.

6. Procédé de détection d'acide nucléique d'un pathogène, notamment d'un virus, dans un échantillon prélevé dans la cavité buccale et/ou les voies respiratoires d'un patient, comprenant les étapes suivantes :
- mise en contact de l'échantillon prélevé avec le milieu de transport selon l'une quelconque des revendications 1 à 3 ;
- transport et/ou stockage dudit échantillon dans le milieu de transport ;
- ultérieurement, détermination, par une méthode basée sur amplification d'acide nucléique, de la présence et/ou de la quantité d'acide nucléique du pathogène dans l'échantillon.

7. Utilisation selon la revendication 4, ou procédé selon la revendication 6, où l'agent pathogène est un coronavirus, de préférence le virus SARS-CoV-2.
